# EUROPEAN PATENT SPECIFICATION

(11) **EP 2 802 309 B1**
(45) Date of publication and mention of the grant of the patent: **30.12.2020**
(21) Application number: 13704496.2
(22) Date of filing: 10.01.2013
(51) Int. Cl.: A61K 8/9794, A61K 8/35, A61K 36/9068, A61Q 19/00, A61P 9/08

(54) **NEW TOPICAL USE OF ZERUMBONE**
NEUE TOPISCHE VERWENDUNG VON ZERUMBON
NOUVELLE UTILISATION TOPIQUE DU ZÉRUMBONE

(30) Priority: 10.01.2012 FR 1250238
(43) Date of publication of application: 19.11.2014
(73) Proprietor: Sederma, 78610 Le Perray en Yvelines (FR)
(72) Inventor: FOURNIAL, Arnaud, 75016 Paris (FR); GRIZAUD, Claire-Marie, 1180 Uccle (BE); MONDON, Philippe, 92120 Montrouge (FR)
(86) International application number: PCT/IB2013/050224
(87) International publication number: WO 2013/105047

(56) References cited:
- MY-A- 140 952
- US-B1- 6 607 756
- N. J. YOB ET AL: "Zingiber zerumbet (L.) Smith: A Review of Its Ethnomedicinal, Chemical, and Pharmacological Uses", EVIDENCE-BASED COMPLEMENTARY AND ALTERNATIVE MEDICINE, vol. 2011, 1 January 2011 (2011-01-01), pages 1-12, XP055040191, ISSN: 1741-427X, DOI: 10.1155/2011/543216
- CHIEN T Y ET AL: "Anti-inflammatory constituents of Zingiber zerumbet", FOOD CHEMISTRY, ELSEVIER LTD, NL, vol. 110, no. 3, 1 October 2008 (2008-10-01), pages 584-589, XP022627511, ISSN: 0308-8146, DOI: 10.1016/J.FOODCHEM.2008.02.038 [retrieved on 2008-02-20]

## Description

### TECHNICAL FIELD

The present invention relates to a new topical use of zerumbone.

The application fields of the invention are ingredients and compositions for the cosmetics, dermo-pharmaceutical and personal care products industries, zerumbone being used for the treatment of the skin and appendages of mammalian, humans or animals.

Zerumbone is a sesquiterpene (3 isoprene units) with a "humulane" type skeleton. His name is the 2E, 6E, 9E-Humulatrien-8-one. Its developped formula is: 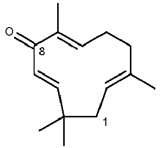 Zerumbone was isolated from roots of *Zingiber zerumbet* (L.) Smith (synonyms: *Amomum zerumbet* L., *Zingiber amaricans* Blume, *Zingiber aromaticum* Val., *Zingiber littorale* Val., *Zingiber zerumbet* (L.) Roscoe), also known as "Ginger Shampoo", "Pinecone Ginger", or "Awapuhi" in Taiwan or "Wild ginger" in China), a plant found in South east Asia. Other plants are said to contain zerumbone as *Alpinia galanga* of Sri Lankan origin or *Syringa pinnafolia.* Zerumbone has anti-inflammatory and anti-radical properties. Effects on cancer in general, especially leukemia, and also effects on the HIV virus are described.

US7588788 describes a nutraceutical composition containing an extract of the roots of *Zingiber zerumbet* (L.) Smith to regulate the immune system and more particularly to prevent and treat allergic disorders.

JP10330243 describes the lightening cosmetic activity of zerumbone extracted from *Zingiber zerumbet* smith.

The present invention aims to propose a new active in particular for the cosmetics, dermo-pharmaceutical and personal care products industries always in demand.

### SUMMARY OF THE INVENTION

To this end, the subject matter of the present invention is a composition comprising zerumbone in a physiologically acceptable medium for a topical treatment of the deficiencies of the microcapillary network of the extracellular matrix. According to the invention, the treatment is a topical skin firming treatment via the protection and repairing of the extracellular matrix (ECM) of the dermis, via an action on elastin fibers and molecules essential to the architecture of the fiber network of the ECM, including collagen, dermatopontine, fibromoduline and cathepsin L.

A composition according to the invention improves the tone of the micro-capillary network of the extracellular matrix.

In particular, the composition of zerumbone is proposed for preventing and treating the heaviness and/or tingling sensations in the lower limbs (treatment of "heavy" and/or "swollen" legs, in particular at the calves and ankles level) and for the treatment of micro-oedema and rednesses (especially in the legs as variculas and in the face especially around the eyes (prevention and treatment of under eyes bags and/or rings)).

The phenomenon of "fatigue, tired or heavy" legs is characterized by a gravity feeling in the legs and fatigability, especially late in the day and during the summer. More than one in three women complains and pregnancy is a period of risk. More than 18 million French people suffer from it. Tired, fatigue or heavy legs are to be taken seriously because they can evolve into real annoying leg pain, which can affect daily life quality and work. Complications can occur: varicose veins, leg ulcers, phlebitis, swelling...

In the normal state, the arteries can supply the tissues and organs of elements essential to their proper functioning, including oxygen. Veins, in turn, allow the return of blood to the heart. In the legs, the blood returns from the bottom upwards thanks to the blood pressure and the tonicity of the vein walls. This blood flow, circulating in the veins and going up back to the heart is called the venous blood return. Valves, acting as small non-return valves are arranged every two to five centimeters. Thus they allow the stream always to flow in the same direction without the possibility of "falling down". So, venous return is always in the same direction. The calf muscles and the compression of the plantar arch also play the role of a pump, especially when walking.

Fatigue, heavy or tired leg syndrome is mainly due to stagnation or slowing down of blood and blood pressure increased in the leg veins. These elements are interrelated in the apparition of clinical symptoms. Vein dilation leading to the formation of varicose veins is associated with loss of tone of the vein wall and hypertension exerted on it. The more the vein is dilated, the more the pressure is increased and the more tone and elasticity are tested. If the veins dilate and the pressure increases, the valves are gradually impaired. They are no more tight, the blood thus stagnates in the lower part of the veins, causing the sensation of tired legs and increase the risk of varicose veins (dilation of small superficial vessels which form a network of blue or purple fine lines on the skin surface of few millimeters to few centimeters long) and varicose veins. The slowing of blood flow and stagnation in turn increases the pressure and dilation. Thus, a real vicious circle takes place. The components of the vein wall are also responsible. The alteration of the tone and elasticity of the vein wall is responsible for the feeling of tired legs. It depends mainly on three of its components:
- Collagen fibers: they can support expansion up to a certain point. Beyond this threshold, they "crack" and tear. Their break dramatically alters the tone of the wall.
- Elastin fibers: in normal physiological, they provide passive feedback to a normal diameter after expansion. Although very durable, they are sensitive to aging. When damaged, the vein does not recover its normal diameter.
- The specialized cells: the contraction of the vein walls is determined by two types of specialized cells: muscle cells and cells of the inner wall in contact with the bloodstream. The latter contain tiny sensors that are very sensitive to changes in pressure, in particular hormones and oxygen level in the blood. When these sensors are activated, they transmit information to the muscle cells of the wall which will then allow either dilation of the vein or contraction as appropriate.

Loss of balance between the different elements of the vein wall (alteration of collagen fibers and/or elastin, activation of wall sensors) at the end lead to a permanent venous dilation which results in a tiredness feeling at the end, followed by pain.

Heavy legs can be caused by:
- Hereditary factors (genetics);
- Heat (especially in summer), in fact temperature promotes vein dilation and the blood tends to stagnate in the legs, which promotes the development of heavy legs;
- Prolonged standing (for instance in commercial jobs) or sitting (long car or airplane trip);
- The gender of the person; it is known that women are more prone to the problem of heavy legs;
- Weight excess (because there is more fat tissue, so more venous tissue irrigation work);
- Age (older people are more concerned about heavy legs);
- Pregnancy and hormonal problems.

Varicose veins are the superficial veins that present a reflux and have lost their drainage function of blood back to the heart and overload the healthy network.

"Matrix-metallo-proteinase" (MMPs) are enzymes that are responsible for cell waste disposal (keratinocytes and fibroblasts). Under the influence of external aggressors such as UV rays, wind, cold, pollution, etc., these MMP's can become more active and attack the dermal extracellular matrix (ECM). In the ECM, are collagen fibers and elastin which are responsible for the tone of the micro-capillaries that remain invisible because of their tone. If the ECM is weakened, the micro-capillaries lose their tone, dilate and become visible on the surface. Under the influence of the same external aggressors, our cells secrete PGE2 and VEGF. PGE2 will be responsible for the expansion of micro-capillaries and VEGF will make them permeable and create a leak of neutrophils which will attack the ECM, which destroys the ECM and expands the micro-capillaries.

It is because the walls of the blood vessels lose their tone that emerge variculas, varicose veins and rosacea.

Through *in vitro* tests, the applicant was able to show that zerumbone actually helps strengthen and preserve the ECM surrounding the micro-capillaries and reduce PGE2 responsible for the dilation of micro-capillary and VEGF permeabilizant of the capillary membranes.

More specifically, it has been shown that zerumbone acts on elastin fibers and molecules essential to the architecture of the fiber network of the ECM, including dermatopontine, fibromoduline and cathepsin L. It has also been shown that zerumbone inhibits the synthesis of MMP1.

The subject matter of the present invention is also the use of zerumbone for a non therapeutical cosmetic topical treatment of skin firming via the protection and repairing of the extracellular matrix (ECM) of the dermis. According to the invention the skin firming treatment acts on elastin fibers and molecules essential to the architecture of the fiber network of the ECM, including collagen, dermatopontine, fibromoduline and cathepsin L.

The topical use is in particular for:
- a general improving of skin firming; and/or
- the treatment of heaviness and/or tingling sensations of the lower limbs; and/or
- the treatment of heavy or tired legs; and/or
- the treatment of eye contour, in particular under eye bags and dark circles.

### DETAILED DESCRIPTION

"Topical use" means an application that is intended to act where it is applied: skin, mucosa, skin appendages. A topical application may be cosmetic and/or dermopharmaceutical.

Zerumbone may be of synthetic origin or brought in the form of a plant extract containing it, more or less purified, in particular an extract of *Zingiber zerumbet* (L.) Smith., *Alpinia galanga* of Sri Lankan origin or *Syringa pinnafolia.*

The present invention preferably uses zerumbone extracted from *Zingiber zerumbet* (L.) Smith (zingiberaceae family and genus *zingiber*).

A plant extract according to the invention can be obtained either directly from the plant or by plant cell culture. Biological material (plant or plant cell culture) is extracted by any method of extraction, eg a conventional aqueous and/or organic solvent (eg alcoholic) extraction or by subcritical or supercritical fluid, microwave or ultra-sounds.

Preferably, according to the invention, subcritical or supercritical fluid extraction is used, in particular by supercritical CO₂, to obtain thus optimal performance and concentration of zerumbone. Liquid CO₂ is an excellent solvent for lipidic products. Inert, it hardly reacts with the molecules extracted and leaves no residue since it is evaporated at the end and captured to be reused.

The extraction can be performed on the whole plant or specific parts of the plant. Preferably according to the invention, the extract is obtained from the roots or rizomes of the plant. According to other features of the invention, zerumbone can be used, especially in cosmetics, in combination with one or more other active ingredients that may for example be selected from the slimming, lightening, anti-redness, UV sunscreens, moisturizers, humectants, exfoliating, anti-aging, anti-wrinkles, volumizing, improving elastic properties, anti-acne, anti-inflammatory, antioxidant, anti-radical, propigmenting or depigmenting, depilatories, anti-regrowth, or promoting hair growth, peptides, vitamins active ingredients, etc.. These active ingredients can be obtained from plant materials, such as plant extracts or plant cell culture products plant or by fermentation.

More specifically, in a cosmetic composition, the extract according to the invention can be combined with at least one of the compounds selected from the compounds of B3 vitamin, the compounds as niacinamide or tocopherol, retinoids compounds, such as retinol, hexamidine, α-lipoic acid, resveratrol or DHEA, peptides, including Pal-KT, Valyl-Tryptophane (VW), N-acetyl-Tyr-Arg-O-hexadecyl ester, Pal-VGVAPG (SEQ ID NO: 1), Pal-KTTKS (SEQ ID NO: 2), Pal-GHK, Pal-KMO₂K and Pal-GQPR (SEQ ID NO: 3), which are widely used active ingredients in topical cosmetic compositions.

Preferably according to the invention, it is proposed to combine zerumbone with a moisturizing ingredient, an active that can bring a freshness feeling, an active further improving the active tone of the dermis, muscles, draining, relaxing, slimming, anti-water-retention, anti-hair regrowth, antioxidant, acting on the heaviness sensations, anti-oedema.

The present invention will be better understood in the light of the following description.

The following examples illustrate the invention without limiting it to the chosen applications.

### Composition preparation for implementing the present invention

The expression "physiologically acceptable medium" means according to the present invention, without limitation, an aqueous or hydroalcoholic solution, a water-in-oil emulsion, an oil-in-water emulsion, a micro-emulsion, an aqueous gel, an anhydrous gel, a serum, a dispersion of vesicles or a powder.

"Physiologically acceptable" means that the compositions are suitable for topical use, in contact with mucous membranes, nails, scalp, hairs and skin of mammals, particularly human, without risk of toxicity, incompatibility, instability, allergic response, and others.

This "physiologically acceptable medium" forms what is commonly called the excipient of the composition.

The effective amount of zerumbone according to the invention, that is to say its dosage, depends on the destination of the topical composition, cosmetic or dermopharmaceutical.

The cosmetic or dermo-pharmaceutical effective amount according to the invention to be administered to treat a disorder or disease and dosage depends on various factors, such as the age, the condition of the patient, the severity of the disorder or disease and the administration mode. An effective amount means a non toxic amount enough to achieve the desired effect.

In a cosmetic composition for the implementing of the present invention, zerumbone, to be present in an effective amount, is generally present in an amount ranging from 0.000001% to 15% based on the total weight of the composition, preferably ranging from 0.00001% to 10%, depending on the destination of the composition and the more or less pronounced desired effect.

All percentages and ratios used herein are by weight of the total composition and all measurements are made at 25°C unless it is otherwise specified.

The choice of the excipient of the composition is made according to the constraints of the zerumbone or the extract containing it (stability, solubility, etc.), and if necessary according to the intended galenic form envisaged afterwards.

Zerumbone is soluble in particular in oil and in an alcohol. It can be incorporated in a composition by means of physiologically acceptable conventional solubilizers, for example and without limiting to this list: ethanol, propanol, isopropanol, propylene glycol, glycerin, butylene glycol, or polyethylene glycol or any combination thereof. It may also be interesting to solubilize the extract using emulsifiers.

A zerumbone extract according to the invention can be used pure or diluted.

The cosmetic compositions are generally prepared by conventional methods well known to one skilled in the art for making topical compositions. Such methods may involve a mixture of ingredients in one or more steps to obtain a uniform state, with or without heating, cooling, etc.

All galenic forms that can contain zerumbone and additional ingredients if present can be used, i.e. solution, emulsion, dispersion, suspension, onguent, cream, lotion, milk, ointment, gel, paste, powder, anhydrous preparation (for example oil for skick, "roll-on"), foam, essence, serum, spray, sprayable formulation, brushable, patch, adhesive material, individually or as a premix or vehicled individually or as a premix in a bound form, incorporated or adsorbed in vectors such as macro-, micro-, or nanocapsules, macro-, micro- or, nanospheres, liposomes, oleosomes or chylomicrons, macro-, micro-, or nanoparticles or macro-, micro or nanosponges, micro- or nanoemulsions, or adsorbed on organic polymer powders, talcs, bentonites, spores or exines, and other inorganic or organic supports, etc.

The composition may be incorporated onto a non-woven or woven material, with natural or synthetic fibers, wool, or any material intended to come into contact with skin and that can be used in clothing, including tights and socks, shorty, day or night underwear, tissues, handkerchiefs or fabric to exert its cosmetic effect via the contact skin/textile and enable continuous topical delivery (cosmetic-textiles).

According to the invention, it is thus also proposed a woven or non-woven fabric comprising zerumbone, derivative and/or analog for use in a cosmetic treatment of insufficiency of the skin capillary network, including the treatment of heaviness and tingling sensations in the inferior limbs. The galenical formulations can enter in different product ranges for personal care and/or beauty products including skin care, cleaning, makeup, cleansing, sunscreen, artificial tanning, pre-shave, shaving or aftershave, moisturizer, humectant, emollient, conditioning, exfoliating, astringent, depilatories or antiperspirant, deodorisant, deodorant, etc.

The CTFA ("International cosmetic ingredient dictionary & handbook" (13th Ed. 2010) published by the "Cosmetic, Toiletry, and Fragrance Association, Inc.", Washington, D.C.) describes a non-limited wide variety of cosmetic and pharmaceutical ingredients conventionally used in the skin care industry that can be used as additional ingredients in the compositions for the present invention, as long as they are physically and chemically compatible with the other ingredients of the composition and especially with the active ingredient of the present invention. Also the nature of these additional ingredients should not unacceptably alter the benefits of the active ingredient of the invention. These additional ingredients can be synthetic or natural such as plants extracts, or come from a bio-fermentation process.

Further skin care actives that are particularly useful combined with the composition can be found in Sederma's commercial literature and on the website www.sederma.fr.

Commercially available actives known for the treatment of heavy legs that can be combined with zerumbone according to the invention include for example: Esculoside™, Phytotonine™, Redulite™ from Sederma, LegActif™ from Provital SA, Phtelene Complex EGX291™ from Greentech, Silidine™ from Greentech, V-tonic™ Gattefossé, Biophytex™ from Cognis-Care Chemicals, GemmoDrain™ from Jan Dekker, Premier red pepper fruit ferment extract™ from Premier Specialities, Rosaceae (blotches) complex™ from Greentech, SHAJIO seed oil™ from Aromtech, Ximilene and Ximenoil™ from Indena, Bladderwrack medulat™, Butcher's broom medulat™, Red vine medulat™ from Greentech.

Commercially available actives acting on water retention can also be mentioned for example Abdoliance™ from Soliance, Adipoless™ de Seppic, Adiporeguline™ from Lucas Meyer, Bioscuptine™ from Silab, Delipidol™ from Solabia, Guaraslim™ from Solabia, Iso-slim Complex™ from Mibelle AG Cosmetics, Lipactive Green Coffee™ from Greentech, Regu slim™ or Regu-shape™ from Pentapharm Ltd, Remoduline from Silab, Ruscogenins C from Indena, Silusyne from Lipotec, Slimactive from Silab, Slimfit™ or Slimmigen™ from Laboratoires Sérobiologiques, Sveltonyl™ from Cognis or Xantalgosil C™ from Exsymol S.A.M. Commercially available actives widely used in cosmetic compositions can also be mentionned as examples: betain, glycerol, Actimoist Bio 2™ (Active organics), AquaCacteen™ (Mibelle AG Cosmetics), Aquaphyline™ (Silab), AquaregulK™ (Solabia), Carciline™ (Greentech), Codiavelane™ (Biotech Marine), Dermaflux™ (Arch Chemicals, Inc), Hydra'Flow™ (Sochibo), Hydromoist L™ (Symrise), RenovHyal™ (Soliance), Seamoss™ (Biotech Marine), Essenskin™ (Sederma), Moist 24™ (Sederma), Argireline™ (commercial name of the acetyl hexapeptide-3 from Lipotec), spilanthol or an *Acmella oleracea* extract known under the trade name Gatuline Expression™, a *Boswellia serrata* extract known under the trade name Boswellin™, Deepaline PVB™ (Seppic), Syn-AKE™ (Pentapharm), Ameliox™, Bioxilift™ (Silab), Juvinity™ (Sederma), Revidrat™ (Sederma), or mixture thereof.

Among plant extracts which can be combined with zerumbone according to the invention, there may more particularly be mentioned extracts of Ivy, in particular English Ivy (*Hedera Helix*), of *Bupleurum chinensis,* of *Bupleurum Falcatum,* of arnica (*Arnica Montana* L), of rosemary (*Rosmarinus officinalis* N), of marigold (*Calendula officinalis*), of sage (*Salvia officinalis* L), of ginseng (*Panax ginseng*), of ginko biloba, of St.-John's-Wort (*Hyperycum Perforatum*), of butcher's-broom (*Ruscus aculeatus* L), of European meadowsweet (*Filipendula ulmaria* L), of big-flowered Jarva tea (*Orthosiphon Stamincus Benth*), of algae (*Fucus Vesiculosus*), of birch (*Betula alba*), of green tea, of cola nuts (*Cola Nipida*), of horse-chestnut, of bamboo, of *Centella asiatica,* of heather, of fucus, of willow, of mouse-ear, of escine, of cangzhu, of *chrysanthellum indicum,* of the plants of the *Armeniacea* genus, *Atractylodis Platicodon, Sinnomenum, Pharbitidis, Flemingia,* of *Coleus* such as C. *Forskohlii,* C. *blumei,* C. *esquirolii,* C. *scutellaroides,* C. *xanthantus* and C. *Barbatus,* such as the extract of root of *Coleus barbatus,* extracts of *Ballote,* of *Guioa,* of *Davallia,* of *Terminalia,* of *Barringtonia,* of *Trema,* of *antirobia, cecropia, argania, dioscoreae* such as *Dioscorea opposita* or Mexican, extracts of *Ammi visnaga,* of *Siegesbeckia,* in particular *Siegesbeckia orientalis,* plant extracts of the family of *Ericaceae,* in particular bilberry extracts (*Vaccinium angustifollium*) or *Arctostaphylos uva ursi, aloe vera,* plant containing sterols (e.g., phytosterol), Manjistha (extracted from plants of the genus *Rubia,* particularly *Rubia Cordifolia*), and Guggal (extracted from plants of the genus *Commiphora,* particularly *Commiphora Mukul*), kola extract, chamomile, red clover extract, *Piper methysticum* extract (*Kava Kava™* from Sederma), *Bacopa monieri* extract (Bacocalmine™ from Sederma) and sea whip extract, extracts of *Glycyrrhiza glabra,* of mulberry, of *melaleuca* (tea tree), of *Larrea divaricata,* of *Rabdosia rubescens,* of *Euglena gracilis,* of *Fibraurea recisa Hirudinea,* of *Chaparral Sorghum,* of sun flower extract, of *Enantia chlorantha,* of Mitracarpe of *Spermacocea* genus, of *Buchu barosma,* of *Lawsonia inermis* L., of *Adiantium Capillus-Veneris* L., of *Chelidonium majus,* of *Luffa cylindrica,* of Japanese Mandarin (*Citrus reticulata Blanco* var. unshiu), of *Camelia sinensis,* of *Imperata cylindrica,* of *Glaucium Flavum,* of *Cupressus Sempervirens,* of *Polygonatum multiflorum,* of *loveyly hemsleya,* of *Sambucus Nigra,* of *Phaseolus lunatus,* of *Centaurium,* of *Macrocystis Pyrifera,* of *Turnera Diffusa,* of *Anemarrhena asphodeloides,* of *Portulaca pilosa,* of *Humulus lupulus,* of *Coffea Arabica,* of *Ilex Paraguariensis,* of *Globularia Cordifolia,* of *Oxydendron arboreum* or of *Ulva Lactuca.*

The compositions of the present invention may include peptides, including, without limitation, the di-, tri-, tetra-, penta-and hexapeptides and their derivatives. According to a particular embodiment, the concentration of the additional peptide, in the composition, ranges from 1x10-7% and 20%, preferably from 1x10-6% and 10%, preferably between 1x10-5% and 5%, by weight.

According to the present invention, the term "peptide" refers to peptides containing 10 amino acids or less, their derivatives, isomers and complexes with other species such as a metal ion (e.g. copper, zinc, manganese, magnesium, and others). The term "peptides" refers to both natural peptides and synthetic peptides. It also refers to compositions that contain peptides which are found in nature, and/or are commercially available.

Suitable dipeptides for use herein include but are not limited to carnosine (beta-AH), YR, VW, NF, DF, KT, KC, CK, KP, KK or TT. Suitable tripeptides for use herein include, but are not limited to RKR, HGG, GHK, GKH, GGH, GHG, KFK, GKH, KPK, KMOK, KMO₂K or KAvaK. Suitable tetrapeptides for use herein include but are not limited to RSRK (SEQ ID NO: 4), GQPR (SEQ ID NO: 5) or KTFK (SEQ ID NO: 6). Suitable pentapeptides include, but are not limited to KTTKS (SEQ ID NO: 7). Suitable hexapeptides include but are not limited to GKTTKS (SEQ ID NO: 8), VGVAPG (SEQ ID NO: 9).

Other suitable peptides for use herein include, but are not limited to lipophilic derivatives of peptides, preferably palmitoyl derivatives, and metal complexes as aforementioned (e.g. copper complex of the tripeptide HGG). Preferred dipeptide derivatives include N-Palmitoyl-beta-Ala-His, N-Acetyl-Tyr-Arg-hexadecylester (Calmosensine™, Idealift™ from Sederma). Preferred tripeptide derivatives include in particular N-Palmitoyl-Gly-Lys-His and N-Palmitoyl-Gly-His-Lys, (Pal-GKH and Pal-GHK from Sederma), the copper derivative of HGG (Lamin™ from Sigma), Lipospondin (N-Elaidoyl-KFK) and its analogs of conservative substitution, N-Acetyl-RKR-NH₂ (Peptide CK+), N-Biot-GHK (from Sederma), Pal-KMO₂K (Sederma) and derivatives thereof. Suitable tetrapeptide derivatives for use according to the present invention include, but are not limited to, N-palmitoyl-GQPR from Sederma (SEQ ID NO: 3), suitable pentapeptide derivatives for use herein include, but are not limited to, N-Palmitoyl-KTTKS (SEQ ID NO: 2) (available as Matrixyl™ from Sederma), N-Palmitoyl-Tyr-Gly-Gly-Phe-X (SEQ ID NO: 10) with X Met or Leu or mixtures thereof. Suitable hexapeptide derivatives for use herein include, but are not limited to, N-Palmitoyl-VGVAPG (SEQ ID NO: 1) and derivatives thereof. The mixture of Pal-GHK and Pal-GQPR (SEQ ID NO: 3) (Matrixyl™ 3000, Sederma) can also be mentioned.

The preferred compositions commercially available containing a peptide or a derivative include Biopeptide-CL™, Maxilip™, Biobustyl™, Procapil™ and Matrixyl™synthe'6™ of Sederma. The compositions commercially available preferred sources of tetrapeptides include Rigin™, Eyeliss™, Matrixyl™ Reloaded and Matrixyl 3000™ which contain between 50 and 500 ppm of Palmitoyl-GQPR (SEQ ID NO: 3) and carrier, proposed by Sederma.

The following commercially available peptides can be mentioned as well as additional active ingredients: Vialox™, Syn-ake™ or Syn-Coll™ (Pentapharm), Hydroxyprolisilane CN™ (Exsymol), Argireline™, Leuphasyl™, Aldenine™, Trylgen™, Eyeseryl™, Serilesine™ or Decorinyl™ (Lipotec), Collaxyl™ or Quintescine™ (Vincience), BONT-L-Peptide™ (Infinitec Activos), Cytokinol™LS (Laboratoires Serobiologiques/Cognis), Kollaren™, IP2000™ or Meliprene™ (Institut Européen de Biologie Cellulaire), Neutrazen™ (Innovations), ECM-Protect™ (Atrium Innovations), Timp-Peptide™ or ECM Moduline™ (Infinitec Activos).

### Cosmetic treatment method

The present invention also provides a method for the treatment of the deficiencies of the cutaneous capillary network, comprising topically applying to the skin and/or its appendages of a subject in need thereof an effective amount of a composition according to the invention comprising zerumbone.

The present invention also provides a method for a cosmetic firming treatment comprising the topical application on the skin of a subject in need thereof of an effective amount of a composition comprising zerumbone in a physiologically acceptable medium.

More particularly, the invention relates to a method for a:
- general firming treatment, and/or
- Treatment of heaviness and/or tingling in the inferior limbs (treatment of "heavy" and/or "swollen" legs, in particular at the level of the calves and ankles), and/or
- The treatment of redness, including variculas; and/or
- The treatment of eye contour (prevention and treatment of under eye bags and dark circles).

The composition according to the invention may be applied locally on areas of the face or the body, in particular the inferior limbs. One of the major advantages of the present invention resides in the ability whenever necessary or desirable to be able to apply local selective "gentle" treatments through this topical, non-invasive method of application.

The topical composition is preferably applied once daily for a period of at least a week, but it can be applied during periods of 2, 4, 8 or 12 weeks. For indication, for a cosmetic face treatment, the European standard dosage of a cream is 2.72 mg/cm²/day/person and for a cosmetic body treatment the European standard dosage of a lotion is 0.5 mg/cm²/day/person.

According to other features, the cosmetic treatment method according to the invention can be combined with one or more other treatment methods targeting the skin such as lumino-therapy, heat or aromatherapy treatments.

According to the invention, devices with several compartments or kits may be proposed to apply the method described above which may include for example and non-restrictively, a first compartment containing a composition comprising zerumbone, and in a second compartment a composition containing another active ingredient, such as providing a feeling of freshness, and/or excipient, the compositions contained in the said first and second compartments in this case being considered to be a combination composition for simultaneous, separate or stepwise use in time, particularly in one of the treatment methods recited above.

### A) Example for obtaining a zerumbone extract

Plant: *Zingiber zerumbet* (L.) Smith.
Part of the plant: dried and crushed roots (rizomes).
Protocol: CO₂ subcritical or supercritical extraction.
Extraction pressure: 70-1000 bars, preferably 70-350 bars.
Temperature: 10-100°C, preferably 20-70°C.
Duration: 24 to 48 hrs.
Purification (filtration, centrifugation, crystallization).

### B) Galenic

Various galenic formulations (cream, gel, lotion, serum, etc.) are described below comprising zerumbone as active molecule to treat deficiencies of the skin capillary network.

Furthermore, these formulations can also contain additional cosmetic active ingredients, the latter coming for each case in support and/or complement of the activity of the active ingredient according to the invention. These ingredients can be of any class according to their(s) function(s), site of application (body, face, neck, chest, hands, etc.), the desired end effect and the target consumer.

Active ingredient of the invention: CO₂ supercritical extract of *Zingiber zerumbet* (L.) Smith, prepared according to above example A, comprising 0.06 % in weight of zerumbone in a butylen glycol matrix.

Additional ingredients: given on a case-by-case as examples, that can be added to the presented formulations, alone or in combination.

### Example 1: Cream gel for « heavy » legs

| **Ingredient** | **%** | **INCI name** |
|---|---|---|
| **Phase A** | | |
| H₂O | Qsp100 | Water |
| Optasense G83 | 0.40 | Carbomer |

| **Phase B** | | |
|---|---|---|
| Crodacol CS 90 | 2.00 | Cetearyl Alcohol |
| Crodamol GTCC | 3.00 | Caprylic/Capric Triglyceride |

| **Phase C** | | |
|---|---|---|
| Butylen glycol | 4.00 | Butylene glycol |
| Phenoxyethanol | 1.00 | Phenoxyethanol |

| **Phase D** | | |
|---|---|---|
| Optasense G82 | 0.20 | Acrylic Acid/Alkylmethacrylate Copolymer |
| DC 200 5 cps | 3.00 | D imethic one |

| **Phase E** | | |
|---|---|---|
| Potassium sorbate | 0.10 | Potassium Sorbate |

| **Phase F** | | |
|---|---|---|
| H₂O | 6.00 | Water |
| NaOH 30% | 0.60 | Sodium Hydroxide |

| **Phase G** | | |
|---|---|---|
| Zerumbone in the form of a *Zingiber zerumbet* (L.) Smith extract | 3.00 | / |

| **Phase H** | | |
|---|---|---|
| Tween 20 | 1.00 | Polysorbate 20 |
| Perfume | 0.10 | Fragrance |

Protocol: Phase A: Sprinkle carbomer in water and let swell without stirring 60 min. Heat phase A at 75°C in a water bath. Weigh phase B, mix and heat at 75°C in a water bath. Weigh phase C and mix. Weigh phase D and mix. Add phase C into phase A under propeller stirring. Add phase B and phase D into phase A+C fast propeller stirring. Add phase E, extemporaneously, in phase A+B+C+D, then mix well under blade stiring for 1 hour. Neutralize with phase F by pouring in the previous phase, mix well. Check the pH around 6. Add phase G at 35°C, mix well. Weigh and add phase H at 35°C, mix well.

### Example 2: massage cream

| **Ingredient** | **%** | **INCI name** |
|---|---|---|
| **Phase A** | | |
| H₂O | Qsp100 | Water |
| Ultrez 10 | 0.25 | Carbomer |

| **Phase B** | | |
|---|---|---|
| Hydrolite-5 | 5.00 | Pentylene Glycol |
| Phenoxyethanol | 1.00 | Phenoxyethanol |
| Keltrol CG-SFT | 0.25 | Xanthan Gum |

| **Phase C** | | |
|---|---|---|
| Brij S2 | 0.40 | Steareth-2 |
| Brij S10 | 1.20 | Steareth-10 |
| Crodafos CES | 4.00 | Cetearyl Alcohol & Dicetyl Phosphate & Ceteth-10 Phosphate |
| Crodamol CSO | 2.50 | Cetearyl Ethylhexanoate |
| BRB CM 56 | 2.00 | Cyclohexasiloxane & Cyclopentasiloxane |
| Crodamol OSU | 7.00 | Diethylhexyl Succinate |
| Crodacol CS 90 | 1.50 | Cetearyl Alcohol |

| **Phase D** | | |
|---|---|---|
| Potassium sorbate | 0.10 | Potassium Sorbate |

| **Phase E** | | |
|---|---|---|
| H₂O | 4.00 | Water |
| NaOH 30 % | 0.40 | Sodium Hydroxide |

| **Phase F** | | |
|---|---|---|
| Zerumbone in the form of a *Zingiber zerumbet* (L.) Smith extract | 3.00 | / |

Protocol: Phase A: Sprinkle carbomer in water, let swell 30 minutes. Heat phase A at 75°C in a water bath. Weigh and mix phase B. Weigh phase C and heat at 75°C in a water bath. Add phase B into phase A under staro stirring. Pour phase C into phase A+B under staro stirring. Mix well. Add phase D, extemporaneously. Neutralize to pH = 6 with phase E below 35°C. Add phase F, mix well.

### Examples of additional ingredients that can be added to this formulation:

REVIDRATE™: moisturising active marketed by Sederma that in particular improves cohesion and hydration of the epidermis. 3% by weight may be added for example to phase C.

BODYFIT™: slimming/firming active ingredient comprising glaucine marketed by Sederma (WO 2004/024695). BODYFIT™ reduces the appearance of cellulite and helps to improve drainage and water distribution in the tissues. 3% by weight may be added for example to phase F of the formulation.

### Example 3: Milk for legs

| **Ingredient** | **%** | **INCI name** |
|---|---|---|
| **Phase A** | | |
| H₂O | Qsp100 | Water |
| Ultrez 10 | 0.15 | Carbomer |

| **Phase B** | | |
|---|---|---|
| Glycerin | 5.00 | Glycerin |
| Phenoxyethanol | 1.00 | Phenoxyethanol |

| **Phase C** | | |
|---|---|---|
| Cithrol GMS AS/NA | 3.50 | Glyceryl Stearate & PEG-100 Stearate |
| Span 60 | 0.70 | |
| Stearic acid | 1.00 | Stearic acid |
| Crodamol ISIS | 2.50 | Isostearyl Isostearate |
| BRB CM 56 | 1.50 | Cyclohexasiloxane & Cyclopentasiloxane |
| Crodamol OP | 10.00 | Ethylhexyl Palmitate |
| Crodacol CS 90 | 0.20 | Cetearyl Alcohol |

| **Phase D** | | |
|---|---|---|
| Potassium sorbate | 0.10 | Potassium Sorbate |

| **Phase E** | | |
|---|---|---|
| H₂O | 4.00 | Water |
| NaOH 30 % | 0.40 | Sodium Hydroxide |

| **Phase F** | | |
|---|---|---|
| Zerumbone in the form of a *Zingiber zerumbet* (L.) Smith extract | 3.00 | / |

Protocol: Phase A: Sprinkle carbomer in water, let swell 30 minutes. Heat phase A at 75°C in a water bath. Weigh and mix phase B. Weigh phase C and heat at 75°C in a water bath. Add phase B into phase A under staro stirring. Pour phase C into phase A+B under staro stirring. Mix well. Add phase D, extemporaneously. Neutralize to pH = 6 with phase E below 35°C. Add phase F, mix well.

### Examples of additional ingredients that can be added to this formulation:

CAPISLOW™: anti-hair regrowth active ingredient marketed by Sederma containing an extract of *Larrea divaricata* rich in nordihydroguaiaretic acid with antiproliferative and anti-inflammatory properties. 3% by weight of CAPISLOW™ may be for example added to phase F of the formulation.

CALMOSENSINE™: soothing active for sensitive skins marketed by Sederma (WO1998/07744) comprising the Tyr-Arg lipo-dipeptide. It reduces discomfort feeliongs. 3.00% by weight of CALMOSENSINE™ can be added to phase F.

### Example 4: Light legs alcoholic serum

| **Ingredient** | **%** | **INCI name** |
|---|---|---|
| **Phase A** | | |
| H₂O | Qsp100 | Water |
| Potassium sorbate | 0.20 | Potassium Sorbate |
| Sodium benzoate | 0.30 | Benzoate de sodium |

| **Phase B** | | |
|---|---|---|
| Organic glycerin | 5.00 | Glycerin |
| Zemea | 5.00 | Propanediol |
| Keltrol CG-SFT | 0.50 | Xanthan Gum |
| Jaguar S | 0.30 | Guar Gum |

| **Phase C** | | |
|---|---|---|
| Natragem S140 | 3.00 | Polyglyceryl-4 Laurate/Sebacate (and) Polyglyceryl-6 Caprylate/Caprate (and) Aqua |
| Organic virgin colza oil | 1.00 | Brassica Campestris Oleifera Oil |
| Covi-Ox T90 | 0.10 | Tocopherol |

| **Phase D** | | |
|---|---|---|
| Ethanol | 10.00 | Alcohol |
| Menthol Cristal | 0.10 | Menthol |

| **Phase E** | | |
|---|---|---|
| H₂O | 0.08 | Water |
| Lactic acid | 0.08 | Lactic Acid |

| **Phase F** | | |
|---|---|---|
| Zerumbone in the form of a *Zingiber zerumbet* (L.) Smith extract | 3.00 | / |

Protocol: Weigh phase A and put it under propeller stiring. Weigh phase B and mix. Add phase B to phase A under slow stirring; mix well for 30 minutes. Weigh phase C and mix. Pour phase C into phase A+B under propeller stirring; mix well under blade stiring. Weigh phase D and add it slowly in the formula under propeller stiring; homogenize under blade stiring. Adjust the pH to 5.00-5.50 with phase E under stirring; mix well. Weigh phase F and add it to the formula, mix well. Check the pH, final pH = 5.50.

### Examples of additional ingredients that can be added to this formulation:

AQUALANCE™: osmoprotecteur moisturising active ingredient marketed by Sederma (WO2009/104118) comprising homarine and erythritol. 3% by weight may be added for example at the end of formulation.

KELISOFT™: anti-hair regrowth active ingredient chelidonine based marketed by Sederma (WO2007/029187). 3% by weight may be added for example at the end formulation.

### Example 5: Lotion

| **Ingredients** | **%** | **INCI name** |
|---|---|---|
| **Phase A** | | |
| H₂O | Qsp100 | Water |

| **Phase B** | | |
|---|---|---|
| Volpo G 26 | 5.00 | Glycereth-26 |
| Phenoxyethanol | 0.80 | Phenoxyethanol |
| Alcohol | 20.00 | Ethanol |

| **Phase C** | | |
|---|---|---|
| Crillet 1 | 2.00 | Polysorbate 20 |
| Crodamol STS | 0.10 | PPG-3 Benzyl Ether Myristate |

| **Phase D** | | |
|---|---|---|
| Potassium sorbate | 0.10 | Potassium Sorbate |

| **Phase E** | | |
|---|---|---|
| Zerumbone in the form of a *Zingiber zerumbet* (L.) Smith extract | 3.00 | |

| **Phase G** | | |
|---|---|---|
| Perfume | 0.10 | Fragrance |

Protocol: Weigh phase A. Weigh phase B and mix. Add phase B into phase A under stirring for 30 minutes. Weigh phase C, mix until having a homogenous blend. Add phase C into phase A+B under stirring. Add phase D in the previous phase. Add phase E under stirring, mix well. Weigh phase F, mix and add to the previous phase; mix well.

### Examples of additional ingredients that can be added to this formulation:

VENUCEANE™: active marketed by Sederma (WO2002/066668) comprising a *Thermus thermophiles* biotechnological extract, that prevents visible signs of photo-aging (spots, wrinkles, dryness ...), protects cell structures from damages caused by UV and strengthens skin integrity. 3% can be added for example to phase E.

HYDRERGY™: active marketed by Sederma (WO2003/02828692) obtained by fermentation of a micro-algae Rhizobium melitoti, which is a long term moisturizing agent and which stimulates the synthesis of ATP. 3% may be added for example to phase E of the formula.

### Example 6: eve contour cream

| **Ingredients** | **%** | **INCI name** |
|---|---|---|
| **Phase A** | | |
| H₂O | Qsp100 | Water |
| Potassium sorbate | qs | Potassium Sorbate |
| Sodium benzoate | qs | Benzoate de sodium |

| **Phase B** | | |
|---|---|---|
| Vegetable butylen glycol | 5.00 | Butylene Glycol |
| Keltrol CG-SFT | 0.60 | Xanthan Gum |
| Kelcogel CG-HA | 0.20 | Gellan Gum |

| **Phase C** | | |
|---|---|---|
| Natragem S140 | 3.00 | Polyglyceryl-4 Laurate/Sebacate (and) Polyglyceryl-6 Caprylate/Caprate (and) Aqua |
| Pripure 3759 | 1.00 | Squalane Vegetal |

| **Phase D** | | |
|---|---|---|
| H₂O | 0.05 | Water |
| Lactic acid | 0.05 | Lactic Acid |

| **Phase E** | | |
|---|---|---|
| Zerumbone in the form of a *Zingiber zerumbet* (L.) Smith extract | 3.00 | |

| **Phase F** | | |
|---|---|---|
| Perfume | 0,10 | Fragrance |

Protocol: Weigh phase A and homogenize.Weigh phase B and mix homogeneously. Add phase B into phase A under propeller stirring. Let swell 1 hour. Weigh phase C, homogenize. Add phase C into phase A+B under strong helix stirring. Leave to homogenize 1 hour under normal propeller stirring. Adjust to pH 5.50 with phase D. Mix 30 minutes under normal propeller stirring. Add phase E in the previous phase, mix well. Add phase F, mix well.

### Examples of additional ingredients that can be added to this formulation:

EYELISS™: active ingredient marketed by Sederma (WO2003/068141) that helps prevent against the appearance of bags under the eyes. It combines three components: hesperidin methyl chalcone reducing capillary permeability, Valyl-Tryptophan (VW) dipeptide which promotes lymphatic circulation and Pal-GQPR lipopeptide that improves firmness, elasticity and reduces inflammation. MATRIXYL™3000: peptide-based anti-wrinkle ingredient marketed by Sederma (WO2005/048968) comprising two matrikines Pal-GHK and Pal-GQPR, which in synergy helps repairing skin damages caused by aging. 3% by weight can be added for example in phase E of the formula.

HALOXYL™: active ingredient for dark circles marketed by Sederma (WO2005/102266). Haloxyl™ combines Pal-GHK and Pal-GQPR matrikines with N-hydroxysuccinimide (NHS) and the chrysin flavonoid. The Pal-GHK and Pal-GQPR reinforce firmness and tone of the eye contour; chrysin and N-hydroxysuccinimide activate the elimination of blood origin pigments responsible for the color of dark circles but also of the local inflammation. 2% by weight of this ingredient may be added to phase E of the formulation.

### C) In vitro and ex vivo studies:

### 1. Reinforcement and preservation of the extracellular matrix (ECM) surrounding the microcapillaries

Strengthening the MEC surrounding the vessels, including limiting its degradation facing various agents can provide better firmness around the vessels (sheathing) and a better circulation.

### a. Action on elastin synthesis, under a nitrosative stress

**Principle:** Explants (41-year-old female, abdominal skin) received a topical cream containing thhe product of the invention (cream according to above example 1 at 18ppm of zerumbone) or a placebo cream (same cream without zerumbone) for two days. At the same time, the culture medium received 18ppm of zerumbone in a liquid form (or its excipient) to potentiate the effects. A NO stress was later applied for 24 hours using an NO donor (NOC-5) integrated in the culture medium. Finally, the product was again applied on the explants and in the culture medium for two days.

The explants were subsequently fixed and elastin fibres were revealed using aVerhoeff stain on 4 µm sections. Counting was done using photos (50 photos/case) by filtering the colours.

**Table 1: Effect of zerumbone on elastin synthesis following a NO stress; n=5**

| **Concentration** | **Elastin (UCA)** | **% Variation; significance** | |
|---|---|---|---|
| **Control without NO** | 6.54 ± 4.5 | Reference 1 | - |
| **Control NO** | 1.18 ± 1.00 | -82%; p<0.01 | Reference 2 |
| **18 ppm of zerumbone** | 2.41 ± 1.40 | - | +104%; p<0.01 |

Results show that the elastin fibres are diminished in number and thickness due to the NO treatment compared to the non stressed control case. The use of zerumbone according to the invention helps maintain the integrity of these fibres thanks to an anti-NO action.

Moreover, a study of normal human fibroblasts (NHF) in culture showed protection of elastin synthesis of +179% (p <0.01) with 5 ppm of the product according to the invention, compared to the control case following the same NO. ELISA test.

### b. Fibromodulin and dermatopontin synthesis

These two molecules although in small amounts in the dermis, play a crucial role in the organization of the extracellular matrix. It is therefore important to preserve and promote their synthesis.

**Principle:** NHFs were put into contact with the product of the invention for two days (fibromodulin) or three days (dermatopontin). The supernatants were then assayed using ELISA tests. At the same time, the number of cells was quantified using the Hoechst method.

**Table 2: Effect of zerumbone on fibromoduline and dermatopontin synthesis, in NHF in culture; n=4**

| **Concentration** | **Fibromodulin (ng/10⁶cell.)** | **% Variation; significance** | |
|---|---|---|---|
| **Control** | 14.0 ± 2.2 | Reference 1 | - |
| **5 ppm of zerumbone** | 51.4 ± 13.4 | - | +268%; p<0.01 |

**Table 3: Effect of zerumbone on dermatopontin synthesis, in NHF culture; n=4**

| **Concentration** | **Dermatopontin (ng/10⁶cell.)** | **% Variation; significance** | |
|---|---|---|---|
| **Control** | 1073 ± 104 | Reference 1 | - |
| **5 ppm of zerumbone** | 2540 ± 361 | - | +137%; p<0,01 |

The synthesis of these two important elements of the dermal matrix is thus facilitated with the product according to the invention.

### c. MMP1 and Cathepsin-S inhibition

**Principle:** NHFs were put into contact with the product of the invention for 24h, and then were radiated with UVA rays to trigger an experimental increase in MMP1 synthesis. The cells once again received the product for 24h and the supernatants were assayed using ELISA tests. At the same time, the number of cells was quantified using the Hoechst method.

**Table 4: Effect of zerumbone on MMP1 synthesis, in NHF in culture, after UVA irradiation; (n=4).**

| **Concentration** | **MMP1 (ng/10⁶cell.)** | **% Variation; significance** | |
|---|---|---|---|
| **Control** | 1406 ± 168 | Reference 1 | - |
| **5 ppm of zerumbone** | 691 ± 225 | - | -51%; p<0.01 |

These results show that zerumbone decreases the synthesis of MMP1 induced by an UVA stress. This inhibition of MMP1 allows strengthening of the ECM. The vessels are better sheathed thanks to a firmer ECM around them.

**Principle:** NHFs were put into contact with the invention product for 24h then were subjected to NO stress. NO donor, NOC-5, was introduced in the culture medium. At the end of a second 24h contact with the zerumbone extrac, a cathepsin-S assay was performed using ELISA. At the same time, the number of cells was quantified using the Hoechst method.

**Table 5: Effect of zerumbone on Cathepsin-1 synthesis, in HNF culture after a NO stress.**

| **Concentration** | **Cathepsine S (pg/10⁶cell.)** | **% Variation; significance** | |
|---|---|---|---|
| **Control** | 405 ± 35 | Reference | - |
| **5 ppm of zerumbone** | 290 ± 40 | - | -34%; p<0.01 |

These results show that the zerumbone decreases the synthesis of cathepsin S induced by nitrosative stress.

### d. Effect of zerumbone on the dermis destruction by proteases

**Principle:** Once prepared, skin explants (40-year-old female, abdominal skin) received an injection of elastase or collagenase that could proteolyse 30 µmol/min of elastin or collagen. In another case, the invention product (titrated at 18ppm of zerumbone) was co-injected with the protease to assess its ability to inhibit the proteolysis of constituents of the dermis, and thus of protectin of the dermis. After five days of incubation at 37°C, the result was observed through a confocal microscope and compared to what was obtained in the case without protease at the same depth (60 µm).

In the cases where a collagenase or elastase stress was applied, it is shown very clearly that the dermis is altered (denaturation of the elastin or collagen microfibers) and disorganized in the control but retains its integrity in the presence of solution containing zerumbone.

Thus these tests show that the zerumbone extract can control the activity and the synthesis of proteases known to modify the structure of the dermis and enable, maintain or protect the synthesis of essential elements of the dermal extracellular matrix.

### 2. Oedema decrease, improvement in circulation and vessel quality

PGE2 is responsible for the dilation of microcapillaries and VEGF will make them permeable and create a neutrophilleak which will attack the ECM, which destroys the ECM and dilates the micro-capillaries.

It is because the walls of the blood vessels lose their tone that emerges variculas, varicose veins and rosacea.

### a) Anti-PGE2 Effect

**Principle:** NHFs at confluence received the compound of the invention in contact with the cells for 48 hours which allowing to evaluate its ability to reduce the basal synthesis of PGE2. The PGE2 assay was performed by ELISA; a quantification of the number of cells was done in parallel.

**Table 6: Effect of zerumbone on PGE2 synthesis in NHF in culture.**

| | **PGE2 (ng / mL / 10E6cell.)** | **% Variation; significance** |
|---|---|---|
| **Control** | 161+/-36 | Reference |
| **5 ppm of zerumbone** | 48+/-10 | -70%; p<0.01 |

Zerumbone greatly rcduccs the synthesis of PGE2.

### b) Anti-VEGF action on activated macrophages

**Principle:** Human macrophage lines (THP-1) obtained by differentiation were activated by a stress model comprised of a bacterial factor, LPS, to induce overproduction of VEGF. This induction was performed both in the presence and in the absence of the product of the invention. 24h later, an assay was performed on the cellular supernatants.

**Table 7: Action of zerumbone on VEGF synthesis in activated macrophage culture; n=3**

| | **VEGF (pg / mL / 106cell.)** | **% Variation; significance** |
|---|---|---|
| **UV Control** | 2004+/- 110 | Reference |
| **5 ppm of zerumbone** | 1177 ± 266 | -41%; p<0.01 |

These results confirm those obtained on HNF. Zerumbone according to the invention reduces VEGF synthesis.

### 3. Reduction of fat storage

### a. Formation of mature and active adipocytes

**Principle:** Mature human adipocytes were formed from immature cells using a hormonal cocktail. One series also received the product of the invention during this phase and the increase in adipose reserves was compared to control cases both visually and by measuring the activity of a fat storage enzyme (G3PDH). A viability test was performed.

**Table 8: Action of zerumbone on the formation of mature adipocytes; n=4**

| **Concentration** | **G3PDH activity mU/ 10⁶ cell** | **% Variation; significance** |
|---|---|---|
| **Control** | 175 ± 9 | Reference 1 |
| **5 ppm of Zerumbone** | 15 ± 3 | -91%; *p*<*0.01* |

These results demonstrate that the product of the invention strongly moderates the maturation of new adipocytes and reduces the storage of new lipids by these normal human adipocytes. This effect, which is otherwise dose-dependant (-39% for the 1ppm equivalent), is accompanied by a significant reduction of lipids that are characteristic of fat storage: cellular triglycerides (see below table).

**Table 9: Action of zerumbone triglyceride production, in mature adipocyte culture; n=4**

| **Concentration** | **Triglycerides µM / 106 cell** | **% Variation; significance** |
|---|---|---|
| **Control** | 4181 +/- 304 | Reference |
| **5 ppm of zerumbone** | 2966 +/- 128 | -29%; *p<0.01* |

### D) In vivo evaluations:

### Principle:

The studies were performed on two panels of women stating that they had problems with heavy legs and mild oedema or water retention. The first study, which was conducted on a panel of 24 volunteers, helped assess decreases in water and fat and blood flow and changes in skin quality. The second study, which was conducted with the help of a phlebologist on a panel of 27 volunteer, assessed the perceived improvement in the legs as well as the circulatory dynamics.

Several complementary methods were associated during this study:
- Analysis of the thickness of ankle adipose tissue by ultrasound
- Analysis of the leg volume by centimetric measurement assisted by a laser
- Analysis of firmness by a Cutometer MPA580
- Analysis of the water quantity by a MoistureMeter-D
- Analysis of the blood flow dynamics by a Laser Doppler Imager
- Clinical assessment by a phlebologist.

Several sites were used for evaluating the effect of zerumbone: from the ankle, which is often symptomatic of heavy leg problems, to the entire leg, through the calf and the thigh.

### Protocol:

Specific inclusion criteria for the two studies: The selected volunteers had to have a sensation of heavy legs on a daily basis and present symptoms of fluid retention/oedema of the ankle. A washout period of one month during which no cosmetic products to reduce heavy leg sensation were used, was requested.

These volunteers had to comply with specific criteria for nutrition and exercise, especially in the hours preceding each appointment.

Moreover, for both of these studies, hormonal consistency was requested for the three months preceding the test and during the test (i.e., no change in contraception, substitution treatment or curative treatment). Only the cosmetic products provided could be used for the duration of the study.

Type of studies and duration: The studies in a single-blind were conducted for the joint evaluation of the product and its placebo, or open-label for a unique evaluation of a product. The studies used non-invasive measures on:
- 24 volunteers with a mean age of 40 [26 to 52 years old] who applied a cream comprising 18 ppm of zerumbone on one leg. The placebo cream was applied contralaterally. Single-blind study.
- 27 volunteers with a mean age of 41 [25 to 50 years old] who solely applied the cream comprising 18ppm of zerumbone on one leg. Open-label study

The cream comprising 18 ppm of zerumbone or the placebo cream was applied by massaging it in twice a day for two months. The study synopsis can be seen in the diagram below.

| T0 | T 1 month | T 2 moths |
|---|---|---|
| Echography | Echography | Echography |
| Measurements (in cm) | Measurements (in cm) | Measurements (in cm) |
| Cutometry | | Cutometry |
| MoistureMeter-D | | MoistureMeter-D |
| Laser Doppler Imager | | LaserDoppler Imager |
| Phlebologist assessment | Phlebologist assessment | Phlebologist assessment |

It should be noted that all the measurements were performed in the afternoon, when the heavy leg phenomenon tends to be at its most intense.

Statistical studies were performed using the Student t test or, if necessary, a nonparametric Wilcoxon test. In both cases, the tests were performed on matched series.

### 1. Analysis of the volume par centimetric laser assisted mesurement

A centimeter measurement was performed at the level of on the thighs, calves and ankles. To obtain a very accurate measurement of the perimeter on these sites, it has been materialized by a laser line (Stanley) perfectly horizontal and whose vertical position was the same at every time measurement. The results are presented in the following table:

**Table 10: Analysis of the leg volume after application of zerumbone according to the invention or its placebo (in ml, 24 volonteers)**

| | Cream comprising zerumbone | | Placebo | |
|---|---|---|---|---|
| | T 1month | T 2months | T 1month | T 2months |
| Delta vs T0 | -92 ml | -230 ml | -44 ml | -78 ml |

The results show that after application of a cream comprising zerumbone, a significant decrease in the volume of the legs is observed. This decrease has already reached 92 ml at 1 month T and becomes significant at 2 months with an overall loss of 230 ml.

### 2. Firmness analysis by a Cutomètre™ MPA580

The Cutometer was used. After an internal study, it appeared that the 6mm probe and the 300mbar aspiration pressure provided the best results usable for this very particular measurement site.

Uv parameter, also called viscous elongation representing the displacement of the fundamental substance within the fiber network, is known to increased with age. Our hypothesis was that the presence of water has made the skin more viscous and that this parameter should be decreased. The results are presented in the table below.

**Table 11: Viscosity variation of the at the level of the ankles after applying the zerumbone according to the invention or its placebo (in mm, 24 volunteers)**

| | **Cream comprising zerumbone** | **Placebo** |
|---|---|---|
| | T 2months | T 2months |
| **% change vs. T0** | -8.8% (p<0.01) | -3.4% (dns) |
| **Significance vs placebo** | p<0.05 | |

The results show that the application of a cream containing zerumbone according to the invention on the ankle improves noticeably the visco-elastic characteristics of the skin. The study of the Uv evolution in a selection of volunteers with the highest Uv at T0 (about half of the panel), shows that the improvement is amplified (-13.3% vs placebo; p <0.01) compared to the general panel.

### 3. Analysis of the adipose layer

This analysis was performed with 10-MHz ultrasonography (DP2200, Mindray).

Each measurement provides mapping for a surface that is 55 mm wide by 65 mm deep and well adapted to measuring adipose tissue thickness, which can vary from 10 to 50 mm, depending on the location in the leg and the type of volunteer.

**Table 12: Thickness change of the adipose tissue at the level of the ankles after applying the zerumbone according to the invention or its Placebo (in mm, 23 volunteers)**

| | **Cream comprising zerumbone** | **Placebo** |
|---|---|---|
| | T 2months | T 2months |
| **% Variation vs. T0** | -5.8% (p<0.01) | -0.5% (dns) |
| **Significance vs placebo** | p<0.01 | |

These results demonstrate the ability of zerumbone according to the invention to reduce the thickness of adipose tissue at the level of the ankle of 6%. The effect, net and significant compared to placebo from the first month, is amplified during the second month of application.

### 4. Analysis of the water content

The MoistureMeter-D (Delfin Technologies) is a device that measures the quantity of water in the skin by measuring impedance.

The study was realised on the ankles, the water content was measured before and after application of the cream comprising zerumbone or its placebo.

**Table 13: Water content change at the level of the ankles after applying the zerumbone according to the invention or its Placebo (in mm, 24 volunteers)**

| | **Cream comprising zerumbone** | **Placebo** |
|---|---|---|
| | T 2months | T 2months |
| **% Variation vs. T0; significance** | -4.6% ; p<0.05 | 2.4% (dns) |
| **% Variation vs placebo; significance** | -7.1% ; p<0.01 | |

A decrease in the quantity of fluid around the ankles was therefore observed after the application of the cream comprising zerumbone, compared with placebo; the difference reaches -7% (p<0.01).This indicates that fluid retention around the ankles was reduced following these applications.

### 5. Analysis of the blood flow dynamics

This measurement can be performed using non-invasive Laser Doppler microcirculation imaging.

A measurement is performed on the volunteer in the sitting position after 5min of rest (basal flow level), and then after an increase in flow caused by controlled dynamic stimulation, i.e., by raising the leg to the horizontal position. Successive measurements in this position are thereafter performed after 1min and 3min.

**Table 14: Variation of the blood flow dynamics at the level of the calves after applicaiton of zerumbone according to the invention or its placebo (in mm, 24 volunteers)**

| | **Flux after 2 months** | | |
|---|---|---|---|
| | **Basal** | **1 min** | **2 min** |
| **Placebo : % Variation vs. Basal** | REF. | +20.1% | +13.6% |
| **Cream comprising zerumbone : % Variation vs. Basal** | REF. | +27.4% | +24.5% |
| **Delta (Product - Placebo); Significance** | | +7.3%; p<0.05 | +10.9%; p<0.01 |

These results show on 24 volunteers that the measured blood flow is increased on the zerumbone according to the invention side of +7.3% (p <0.05) compared to the placebo side after the first minute and by nearly +11% (p <0.01) after the third minute.

### 6. Assessment by a phlebologist

In order to confirm the quantitative measurements, a clinical assessment with the help of a phlebologist was implemented on 27 volunteers. The volunteers were examined and questioned while lying down on an examination table after a 20-minute adjustment period in a controlled atmosphere (temperature 21 + 1°C, barometer 45% ± 5%).

The phlebologist subsequently assessed the intensity of varicosities that were not very prominent, but visible nonetheless as "spider veins" as well as their colouration. The assessment of the intensity of varicosities was based on their abundance.

These results show that the number of volunteers with intense varicosities diminished by 12% compared with the phlebologist's score at T0. At the same time, the percentage of people with bluish varicosities dropped by 30% from T0 to T2 months. Moreover, the clinical assessment of these visible factors was completed by an assessment of what the volunteers felt. The score ranged from 0 (no sensation) to 10 (strong sensation). The same type of score was used to assess the heavy leg sensation. These data show that the sensation of pain declined significantly by 14% between the two points of time (p<0.06; vs. T0). In parallel, the phlebologist noted that the sensation of heavy legs diminished in intensity in volunteers (-28%; p<0.01, vs. T0). Other data (not shown) also demonstrated an 11% decrease in the number of volunteers feeling discomfort throughout the day. This clinical assessment, performed by a phlebologist, shows the direct impact on the improvement of the quality of life of the volunteer, both in terms of sensation and appearance.

These results confirm those obtained in metrology.

## Claims

1. Use of zerumbone for a non therapeutical cosmetic topical treatment of skin firming via the protection and repairing of the extracellular matrix (ECM) of the dermis.

2. Use according to claim 1, wherein the skin firming treatment acts on elastin fibers and molecules essential to the architecture of the fiber network of the ECM, including collagen, dermatopontine, fibromoduline and cathepsin L.

3. Use according to claim 1 or 2, for the treatment of heaviness and/or tingling sensations of the lower limbs.

4. Use according to claim 3, for the treatment of legs.

5. Use according to claim 1 or 2, for the treatment of eye contour, in particular under eye bags and dark circles.

6. Woven or non-woven fabric material comprising zerumbone suitable for a use according to anyone of claims 1 to 5.

## Patentansprüche

1. Verwendung von Zerumbon für eine nicht-therapeutische kosmetische topische Behandlung zur Hautstraffung mittels des Schutzes und des Reparierens der extrazellulären Matrix (ECM) der Dermis.

2. Verwendung nach Anspruch 1, wobei die Hautstraffungsbehandlung auf Elastinfasern und Molekülen wirkt, die für die Architektur des Fasernetzwerks der ECM wesentlich sind, einschließlich Kollagen, Dermatopontin, Fibromodulin und Cathepsin L.

3. Verwendung nach Anspruch 1 oder 2 zur Behandlung von Schwere- und/oder Kribbelgefühlen der unteren Gliedmaßen.

4. Verwendung nach Anspruch 3 zur Behandlung von Beinen.

5. Verwendung nach Anspruch 1 oder 2 zur Behandlung der Augenkontur, insbesondere der Tränensäcke und Augenringe.

6. Gewebe oder Vliesmaterial, umfassend Zerumbon, das zur Verwendung nach einem der Ansprüche 1 bis 5 geeignet ist.

## Revendications

1. Utilisation de zerumbone pour un traitement topique cosmétique non-thérapeutique de renforcement de la peau via la protection et la réparation de la matrice extracellulaire dermique (MEC).

2. Utilisation selon la revendication 1, dans laquelle le traitement de renforcement de la peau présente une action sur les fibres d'élastine et sur des molécules essentielles à l'architecture du réseau de fibres de la MEC, notamment la dermatopontine, la fibromoduline et la cathepsine L.

3. Utilisation selon la revendication 1 ou 2, pour le traitement des sensations de lourdeur ou de fourmillement des membres inférieurs.

4. Utilisation selon la revendication 3, pour le traitement des jambes.

5. Utilisation selon la revendication 1 ou 2 pour le traitement du contour des yeux, notamment des cernes et des poches.

6. Matériau tissé ou non-tissé comprenant de la zerumbone adapté à l'utilisation selon l'une des revendications 1 à 5.


### SEQUENCE LISTING

<110> SEDERMA
   FOURNIAL, ARNAUD
   GRIZAUD, CLAIRE-MARIE
   MONDON, PHILIPPE
<120> NEW TOPICAL USE OF ZERUMBONE
<130> SEXY PCT
<150> FR1250238
   <151> 2012-01-10
<160> 10
<170> PatentIn version 3.5
<210> 1
   <211> 6
   <212> PRT
   <213> artificial sequence
<220>
   <223> synthetic peptide
<220>
   <221> MOD_RES
   <222> (1)..(1)
   <223> amidation by a Palmitoyl chain on the N terminal end
<400> 1 
Val Gly Val Ala Pro Gly
1               5
<210> 2
   <211> 5
   <212> PRT
   <213> artificial sequence
<220>
   <223> synthetic peptide
<220>
   <221> MOD_RES
   <222> (1)..(1)
   <223> amidation by a Palmitoyl chain on the N terminal end
<400> 2 
Lys Thr Thr Lys Ser
1               5
<210> 3
   <211> 4
   <212> PRT
   <213> artificial sequence
<220>
   <223> synthetic peptide
<220>
   <221> MOD_RES
   <222> (1)..(1)
   <223> amidation by a Palmitoyl chain on the N terminal end
<400> 3 
Gly Gln Pro Arg
1
<210> 4
   <211> 4
   <212> PRT
   <213> artificial sequence
<220>
   <223> synthetic peptide
<400> 4 
Arg Ser Arg Lys
1
<210> 5
   <211> 4
   <212> PRT
   <213> artificial sequence
<220>
   <223> synthetic peptide
<400> 5 
Gly Gln Pro Arg
1
<210> 6
   <211> 4
   <212> PRT
   <213> artificial sequence
<220>
   <223> synthetic peptide
<400> 6 
Lys Thr Phe Lys
1
<210> 7
   <211> 5
   <212> PRT
   <213> artificial sequence
<220>
   <223> synthetic peptide
<400> 7 
Lys Thr Thr Lys Ser
1               5
<210> 8
   <211> 6
   <212> PRT
   <213> artificial sequence
<220>
   <223> Synthetic peptide
<400> 8 
Gly Lys Thr Thr Lys Ser
1               5
<210> 9
   <211> 6
   <212> PRT
   <213> artificial sequence
<220>
   <223> synthetic peptide
<400> 9 
Val Gly Val Ala Pro Gly
1               5
<210> 10
   <211> 5
   <212> PRT
   <213> artificial sequence
<220>
   <223> synthetic peptide
<220>
   <221> MOD_RES
   <222> (1)..(1)
   <223> amidation by a Palmitoyl chain on the N terminal end
<220>
   <221> MISC_FEATURE
   <222> (5)..(5)
   <223> Xaa being either a Methionine M or a Leucine L.
<400> 10

Tyr Gly Gly Phe Xaa
1               5